# EUROPEAN PATENT APPLICATION

(11) **EP 4 809 953 A1**
(43) Date of publication of application: **23.09.2026**
(21) Application number: 24891247.9
(22) Date of filing: 01.11.2024
(51) Int. Cl.: A61B 18/14, A61L 31/10

(54) **SURGICAL ELECTRODE HAVING SURFACE TREATMENT COATING**

(30) Priority: 13.11.2023 JP 2023193045
(71) Applicant: Nihon Parkerizing Co., Ltd., Tokyo 103-0027 (JP)
(72) Inventor: FUJITA, Ryuichi, Tokyo 103-0027 (JP); UCHIDA, Junichi, Tokyo 103-0027 (JP); YOROZU, Takayuki, Tokyo 103-0027 (JP)
(74) Representative: Watermeyer, Nicholas
(86) International application number: PCT/JP2024/039000
(87) International publication number: WO 2025/105207

(57) **Abstract**

An object is to provide a surgical electrode of an electrosurgical instrument used for surgery of a living tissue, the surgical electrode having a coating to which carbides of the living tissue and the like are unlikely to adhere and which has excellent adhesion, by incorporating a surface treatment coating showing a specified infrared absorption spectrum, to an end portion of the surgical electrode. In particular, even in both a condition where blood is much and a condition where protein and/or lipid are/is much, good burn resistance is exhibited. The present disclosure relates to a surgical electrode of an electrosurgical instrument used for surgery of a living tissue, wherein the surgical electrode comprises an end portion capable of emitting a high frequency, the end portion has a surface treatment coating, and the surface treatment coating has a peak (α) in a range of 950 cm⁻¹ to 1060 cm⁻¹, a peak (β) in a range of 720 cm⁻¹ to 830 cm⁻¹, and a peak (γ) in a range of 450 cm⁻¹ to 495 cm⁻¹ in an infrared absorption spectrum, and a ratio (β_{A}/α_{A}) of a peak intensity (β_{A}) of the peak β to a peak intensity (α_{A}) of the peak α is 0.770 to 1.040.

## Description

### TECHNICAL FIELD

The present invention relates to a surgical electrode having a surface treatment coating, which can be preferably used in an electrosurgical instrument that is used for surgery of a living tissue as a medical device.

### BACKGROUND ART

In surgical procedures, an electrosurgical instrument (so called electric cautery), which can perform hemostasis (coagulation) and incision by discharging a high-frequency electric current generated by its main body from a surgical electrode to a living tissue, is indispensable. The use of an electric cautery is known to have a problem of causing an "eschar" in which carbides of a living tissue and the like adhere to the tip of the electric cautery and, with regard to this problem, there has been proposed a method of mass-producing plural electrodes that can each be connected to an appropriate electrical power source for surgical procedure, which method is characterized by including the steps of: preparing an electroconductive stock material that has a shape and dimensions for forming plural electrode blanks; coating at least a part of the stock material with a non-stick layer; and forming plural coated electrode blanks (see Patent Document 1).

### RELATED ART DOCUMENT

### PATENT DOCUMENT

[Patent Document 1] Japanese Patent Laid-Open No. 2000-333968

### SUMMARY OF THE INVENTION

### PROBLEMS TO BE SOLVED BY THE INVENTION

However, the technology disclosed in Patent Document 1 was found to have a problem that the coated non-stick layer is damaged by Joule heat and discharge voltage associated with a high-frequency electric current released from the tip of an electric cautery, and the non-stick layer is consequently peeled off or eliminated. In particular, burn easily changes depending on the part of incision (a case where blood is much, a case where protein and/or lipid are/is much, and/or the like), and interferes with stable surgery.

The present invention solves this problem, and an object of the present invention is to provide a surgical electrode of an electrosurgical instrument used for surgery of a living tissue, the surgical electrode having a coating to which carbides of the living tissue and the like are unlikely to adhere and which has excellent adhesion. In particular, even in both a condition where blood is much and a condition where protein and/or lipid are/is much, good burn resistance is exhibited.

### MEANS FOR SOLVING THE PROBLEMS

The present inventors intensively studied to solve the above-described problem and consequently discovered that a surgical electrode having a coating to which carbides of a living tissue and the like are unlikely to adhere even under various conditions and which has excellent adhesion can be provided by incorporating a surface treatment coating showing a specified infrared absorption spectrum, to an end portion of the surgical electrode, thereby completing the present invention.

That is, the present invention can encompass the following.
<1> A surgical electrode of an electrosurgical instrument used for surgery of a living tissue,
   wherein the surgical electrode comprises an end portion capable of emitting a high frequency,
   the end portion has a surface treatment coating, and
   the surface treatment coating has a peak (α) in a range of 950 cm⁻¹ to 1060 cm⁻¹, a peak (β) in a range of 720 cm⁻¹ to 830 cm⁻¹, and a peak (γ) in a range of 450 cm⁻¹ to 495 cm⁻¹ in an infrared absorption spectrum, and a ratio (β_{A}/α_{A}) of a peak intensity (β_{A}) of the peak β to a peak intensity (α_{A}) of the peak α is in a range of 0.770 to 1.040.
<2> The surgical electrode according to <1>, wherein the surface treatment coating comprises a silicone resin (A), and the silicone resin (A) comprises a constituting unit composed of a D unit and a constituting unit composed of a T unit.
<3> The surgical electrode according to <2>, wherein the surface treatment coating comprises a silicon-comprising inorganic oxide (B).
<4> The surgical electrode according to any of <1> to <3>, wherein the end portion further comprises a base coating, which is formed from a surface treatment agent (Y) comprising an amino group-comprising compound (D).

### ADVANTAGEOUS EFFECTS OF THE INVENTION

According to the present invention, a surgical electrode of an electrosurgical instrument used for surgery of a living tissue, in which carbides of the living tissue and the like are unlikely to adhere even under various conditions and high performance corresponding to excellent adhesion is exhibited.

### BRIEF DESCRIPTION OF THE DRAWINGS

[FIG. 1] The figure shows a schematic drawing that illustrates one example (blade electrode) of a surgical electrode (electric cautery).
[FIG. 2] The figure shows a schematic drawing that illustrates one example (loop-type electrode) of a surgical electrode (electric cautery).
[FIG. 3] The figure shows a schematic drawing that illustrates one example (ball-type electrode) of a surgical electrode (electric cautery).
[FIG. 4] The figure shows a schematic drawing that illustrates one example (needle-type electrode) of a surgical electrode (electric cautery).
[FIG. 5] The figures show schematic drawings that illustrate an example of a surgical electrode (laparoscope). (a) illustrates a wire L-shaped hook type, (b) illustrates a straight spatula type, (c) illustrates a wire J-shaped hook type, and (d) illustrates a syringe type.
[FIG. 6] The figure shows a schematic drawing that illustrates one example (bipolar-type) of a surgical electrode (electric cautery).
[FIG. 7] The figures show schematic drawings that illustrate a formation example of a base coating on a surgical electrode.
[FIG. 8] The figures show schematic drawings that illustrate formation examples of a base coating and a surface treatment coating on a surgical electrode.

### DESCRIPTION OF EMBODIMENTS

The surgical electrode comprising a surface treatment coating according to one embodiment of the present invention is used for surgery of a living tissue and comprises a specified surface treatment coating on or over the surface of an end portion of the surgical electrode. The surgical electrode may further comprise a passivation coating and/or an oxide coating of iron between the end portion of the surgical electrode and the surface treatment coating, but it is also acceptable not to have these coatings.

### <Surgical Electrode>

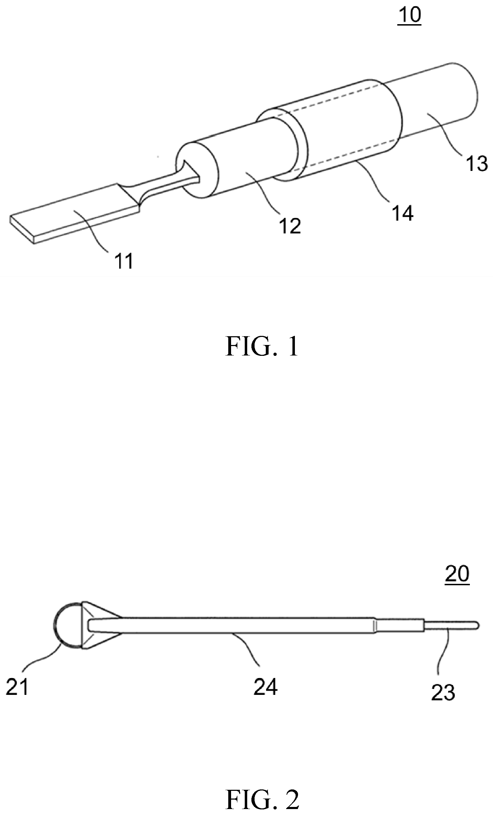

The surgical electrode is an electrode to be fitted onto a tip of an electrosurgical instrument such as a so-called electric cautery in a detachable manner, and is capable of performing hemostasis (coagulation) and incision of a living tissue by emitting a high frequency to the living tissue from an end portion of the electrode. The surgical electrode is composed of an electroconductive material. More specifically, examples include iron-based metal materials, zinc-plated metal materials, aluminum-based metal materials, magnesium-based metal materials, nickel-based metal materials, titanium-based metal materials, zirconium-based metal materials, copper-based metal materials, tin-based metal materials, tungsten-based metal materials, chromium-based metal materials, manganese-based metal materials, molybdenum-based metal materials, and cobalt-based metal materials, and the like, but stainless steel is more preferable. Typical examples of an electrosurgical instrument to which the surgical electrode is fitted include electric cauteries such as monopolar cauteries and bipolar cauteries, and laparoscopes. FIG. 1 is a schematic drawing that illustrates one example of the surgical electrode.

FIG. 1 illustrates one example of a blade-type surgical electrode whose end portion is in a plate form.

A surgical electrode 10 is a member which can be attached to and detached from an electrosurgical instrument main body, which is not illustrated. The surgical electrode 10 is constituted by: an electrical connection portion 13, which is electrically connected to the electrosurgical instrument main body; an end portion 11, which is brought into close contact with a living tissue and from which a high frequency is emitted; and an intermediate portion 12, which connects the electrical connection portion 13 and the end portion 11.

### <End Portion>

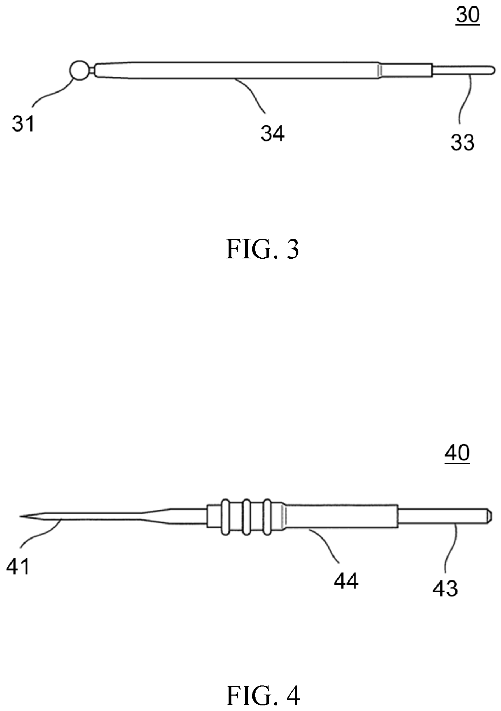

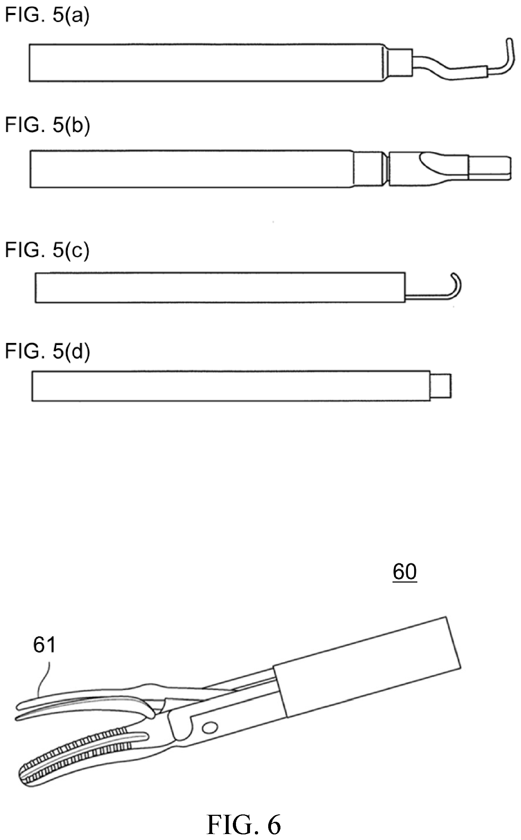

The end portion 11 is a part that emits a high frequency by being brought close contact with a living tissue. The shape of the end portion is not particularly restricted, and in addition to the end portion 11 of the blade-type surgical electrode 10 illustrated in FIG. 1, there exist an end portion 21 of a loop-type surgical electrode 20 illustrated in FIG. 2; an end portion 31 of a ball-type surgical electrode 30 illustrated in FIG. 3; and an end portion 41 of a needle-type surgical electrode 40 illustrated in FIG. 4, and the like, and these surgical electrodes are used as tip electrodes of electric cauteries. In addition to these, there are surgical electrodes fitted to a laparoscope, such as a wire L-shaped hook-type electrode illustrated in FIG. 5(a), a straight spatula-type electrode illustrated in FIG. 5(b), a wire J-shaped hook-type electrode illustrated in FIG. 5(c), and a syringe-type electrode illustrated in FIG. 5(d). The above-described end portions are each an end portion of a monopolar-type surgical electrode; however, they may each be an end portion of a bipolar-type surgical electrode. FIG. 6 illustrates one example of an end portion 61 of a bipolar-type surgical electrode 60.

The end portion 11 may be subjected to roughening treatment on at least a part (e.g., a part where the surface treatment coating is formed) of the surface of the electroconductive material, but it may also be one that has not been subjected to roughing treatment. Examples of a method for performing the roughening treatment include, for example, an etching method using a solution (e.g., an acidic solution or an alkaline solution), a grinding method, a plasma treatment method, and a corona discharge treatment method, and the like, but are not limited to these. These treatments may be performed singly, or two or more thereof may be performed in combination. The surface roughness of the end portion 11 is preferably in a range of 0.05 µm to 0.39 µm, more preferably in a range of 0.08 µm to 0.25 µm, particularly preferably in a range of 0.10 µm to 0.18 µm in terms of arithmetic average roughness Ra. Here, in this specification, "surface roughness" means line roughness, and the above-described Ra is a value measured by a contact-type surface roughness meter.

### <Electrical Connection Portion>

The electrical connection portion 13 is the portion in the surgical electrode 10 that is electrically connected to a main body of an electrosurgical instrument. The electrical connection portion 13 can be attached to or detached from the main body of the electrosurgical instrument, and is usually configured such that it can be fitted with the main body of the electrosurgical instrument by a mating structure or the like. The electrical connection portion is also composed of an electroconductive material, which may be the same as or different from that of the end portion 11.

### <Intermediate Portion >

The intermediate portion 12 is a member which connects the end portion 11 to the electrical connection portion 13. The intermediate portion 12 is necessary to be composed of an electroconductive material for electrical conduction to the end portion 11; however, the shape, the length and the like are not particularly restricted.

The intermediate portion 12 may comprise a cover 14. The cover 14 is a cured product of a composition comprising an insulating resin. Further, the size, the thickness, the shape and the like of the cover 14 are not particularly restricted as long as the intermediate portion 12 and the cover 14 are in contact with each other.

### <Surface Treatment Coating>

The surface treatment coating according to the present embodiment has an absorption maximum (hereinafter, referred to as absorption peak) at a predetermined number in an infrared absorption spectrum. In the surface treatment coating, an absorption peak α in a range of 950 cm⁻¹ to 1060 cm⁻¹ (hereinafter, referred to as "peak α"), an absorption peak β in a range of 720 cm⁻¹ to 830 cm⁻¹ (hereinafter, referred to as "peak β"), and an absorption peak γ in a range of 450 cm⁻¹ to 495 cm⁻¹ (hereinafter, referred to as "peak γ") are present. The ratio (β_{A}/α_{A}) of the peak intensity (β_{A}) of the peak β to the peak intensity (α_{A}) of the peak α in the surface treatment coating is preferably in a range of 0.770 to 1.040, more preferably in a range of 0.830 to 1.034, further preferably in a range of 0.950 to 1.023. The ratio β_{A}/α_{A} of the peak intensities is in the above-described range to allow good burn resistance and adhesion to be obtained.

The peak α indicates, for example, the presence of Si-O stretching vibration. The peak β indicates, for example, the presence of Si-C stretching vibration. The peak γ indicates, for example, the presence of an inorganic solid comprising a Si-O bond.

The ratio (γ_{A}/α_{A}) of the peak intensity (γ_{A}) of the peak γ to the peak intensity α_{A} of the peak α is preferably in a range of 0.005 to 0.135. The ratio (γ_{A}/β_{A}) of the peak intensity (γ_{A}) of the peak γ to the peak intensity β_{A} of the peak β is preferably 0.005 to 0.180.

A method for analyzing the infrared absorption spectrum of the surface treatment coating is not particularly restricted, and examples include an ATR method as one infrared spectroscopy. In the case of analysis by an ATR method, an apparatus is, for example, FT-IR (Spectrum Two) manufactured by Perkin Elmer, to which auxiliary equipment for ATR measurement (GladiATR^{™}) is attached.

A method for forming the surface treatment coating according to the present embodiment is not restricted as long as desired absorption intensities for the peak α, the peak β, and the peak γ are obtained, but the surface treatment coating is formed by, for example, contacting a surface treatment agent (X) with or over the surface of an end portion of the surgical electrode. In a case where the end portion of the surgical electrode is provided with a base coating, the surface treatment coating is formed by contacting a surface treatment agent (X) with the surface of the base coating. The surface treatment agent (X) may be contacted with a part or the entirety of the end portion or the base coating.

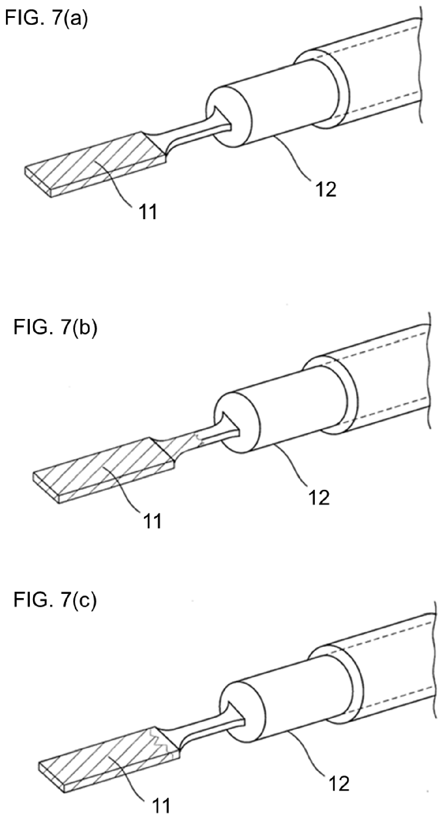

The surface treatment coating may be formed on the entire surface of the end portion or on a part of the end portion. In the case of a blade-type surgical electrode, examples of the "part" include the blade part of the end portion, and the flat part of the end portion. In the present specification, "flat part" refers to a part having the largest area in the blade part of the end portion 11 illustrated in FIGS. 7(a) to 7(c).

One example of a more preferred embodiment is the end portion provided with a base coating, which is formed from a surface treatment agent (Y) comprising an amino group-comprising compound (D).

In those parts where the surface treatment coating is not formed, only the base coating may be formed. For example, in the case of a blade-type surgical electrode, the base coating may be formed on the entire surface of the blade part, and the surface treatment coating may be formed partially on the base coating. Further, in the end portion 11, only the base coating or both the base coating and the surface treatment coating may be formed on a part or the entirety of the surface of the end part (hereinafter, referred to as "intermediate connecting part") on the distal end of the intermediate portion 12.

### <Surface Treatment Agent (X)>

The surface treatment agent (X) according to the present embodiment preferably comprises a silicone resin (A) and a silicon-comprising inorganic oxide (B), from the viewpoint of formation of a surface treatment coating which satisfies the requirements of the peak intensities in the infrared absorption spectrum.

### <Silicone Resin (A)>

The silicone resin (A) is not particularly restricted as long as it has an organopolysiloxane structure which comprises plural siloxane bonds and in which an organic group is bound to silicon (Si).

From the viewpoint that the surface treatment coating satisfies the above-described requirements of the peak intensities in the infrared absorption spectrum, the silicone resin (A) preferably comprises a silicone resin (A2) comprising an organopolysiloxane structure (M unit or D unit) having at least one or two organic groups bound to Si in one molecule and a silicone resin (A1) comprising an organopolysiloxane structure (T unit or Q unit) having at least three or four organic groups bound to Si in one molecule. In particular, the silicone resin (A) preferably comprises a rubber-like silicone resin (A2) comprising the D unit and a resin-like silicone resin (A1) comprising the T unit. The position at which each organic group is bound is not particularly restricted, and each organic group may be bound to a main chain, a side chain, or a terminal.

The silicone resin (A) may be a homopolymer comprising the above-described organopolysiloxane structure, a mixture of a homopolymer comprising the above-described organopolysiloxane structure and a homopolymer copmrising a polysiloxane structure, or a copolymer (a block copolymer or a graft polymer) comprising the above-described organopolysiloxane structure and a polysiloxane structure. The silicone resin (A) may be of an addition type or a condensation type. Moreover, the silicone resin (A) may be any of a thermosetting type, a room temperature-curable type (RTV), and a UV-curable type.

Examples of the organic group bound to Si in the organopolysiloxane structure include saturated hydrocarbon groups, unsaturated hydrocarbon groups, halogenated alkyl groups, and epoxycycloalkyl group and the like, but these are not limited thereto. Examples of the saturated hydrocarbon groups include linear or branched alkyl groups and cycloalkyl groups and the like, but these are not limited thereto. Further, examples of the unsaturated hydrocarbon groups include linear or branched alkenyl groups; cycloalkenyl groups; cycloalkenylalkyl groups; and aryl groups and the like, but these are not limited thereto. The organic group bound to Si is preferably an unsaturated hydrocarbon group, more preferably an alkenyl group, particularly preferably a vinyl group or a hexenyl group.

Examples of the halogenated alkyl groups include chloromethyl group, 3-chloropropyl group, 1-chloro-2-methylpropyl group, and 3,3,3-trifluoropropyl group and the like. Examples of the epoxycycloalkyl groups include epoxycyclopentyl group, epoxycyclohexyl group, and epoxycyclooctyl group and the like. Examples of the linear or branched alkyl groups include methyl group, ethyl group, propyl group, isopropyl group, butyl group, isobutyl group, pentyl group, hexyl group, heptyl group, octyl group, nonyl group, and decyl group and the like. Examples of the cycloalkyl groups include cyclopentyl group and cyclohexyl group and the like. Examples of the linear or branched alkenyl groups include vinyl group, 1-propenyl group, allyl group, isopropenyl group, 1-butenyl group, 2-butenyl group, pentenyl group, and hexenyl group and the like. Examples of the cycloalkenyl groups include cyclopentenyl group and cyclohexenyl group and the like. Examples of the cycloalkenylalkyl groups include cyclopentenylethyl group, cyclohexenylethyl group, and cyclohexenylpropyl group and the like. Examples of the aryl groups include phenyl group and the like.

The polysiloxane structure is not particularly restricted as long as it is different from the above-described organopolysiloxane structure, and examples thereof include a polysiloxane structure that comprises at least two or more hydrogen atoms bound to Si in one molecule, and a polysiloxane structure that comprises at least two or more alkoxy groups bound to Si in one molecule, and the like. Examples of the alkoxy groups include methoxy group, ethoxy group, propoxy group, and butoxy group and the like. The alkoxy groups may be each linear or branched.

The above-described various silicone resins (A) may be used singly, or in combination of two or more kinds thereof, in the preparation of the surface treatment agent (X). One example of the preferred embodiments of the silicone resin (A1) comprising the T unit is a mixture of a polymer comprising an organopolysiloxane structure that comprises at least two or more unsaturated hydrocarbon groups bound to Si in one molecule and a polymer comprising a polysiloxane structure that comprises at least two or more hydrogen atoms bound to Si in one molecule.

Examples of the polymer comprising an organopolysiloxane structure that comprises at least two or more unsaturated hydrocarbon groups bound to Si in one molecule include a dimethyl polysiloxane having dimethylvinyl siloxy groups at both terminals of its molecular chain, a dimethyl siloxane-methylphenyl siloxane copolymer having dimethylvinylsiloxy groups at both terminals of its molecular chain, a dimethyl siloxane-methylvinyl siloxane copolymer having dimethylvinylsiloxy groups at both terminals of its molecular chain, a dimethyl siloxane-methylvinyl siloxane copolymer having trimethylsiloxy groups at both terminals of its molecular chain, a dimethyl siloxane-methylvinyl siloxane-methylphenyl siloxane ternary copolymer having trimethylsiloxy groups at both terminals of its molecular chain, a dimethyl siloxane-methylvinyl siloxane copolymer having silanol groups at both terminals of its molecular chain, and a methylvinyl polysiloxane having silanol groups at both terminals of its molecular chain, and the like. In addition, polymers in which some of the methyl groups of various homopolymers, copolymers and ternary copolymers are substituted with: alkyl groups other than a methyl group, such as an ethyl group and a propyl group and the like; or halogenated alkyl groups, such as a 3,3,3-trifluoropropyl group and a 3,3,3-trichloropropyl group and the like, can also be exemplified. A mixture of two or more selected from the above-described homopolymers, copolymers and ternary copolymers may be used for the preparation of the surface treatment agent (X).

The polymer comprising a polysiloxane structure that comprises at least two or more hydrogen atoms bound to Si in one molecule is not particularly restricted, and examples thereof include organohydrogen polysiloxanes having a linear, cyclic, branched or three-dimensional network structure which comprises at least two or more SiH groups, in each of which a hydrogen atom is bound to Si, in one molecule and has repeating diorganosiloxane structure as a main chain and whose molecular chains are capped with triorganosiloxy groups at both terminals. More specific examples include a methyl hydrogen polysiloxane having trimethylsiloxy groups at both terminals of its molecular chain, a dimethyl siloxane-methyl hydrogen siloxane copolymer having trimethylsiloxy groups at both terminals of its molecular chain, a methyl hydrogen polysiloxane having silanol groups at both terminals of its molecular chain, a dimethyl siloxane-methyl hydrogen siloxane copolymer having silanol groups at both terminals of its molecular chain, a dimethyl polysiloxane having dimethyl hydrogen siloxy groups at both terminals of its molecular chain, a methyl hydrogen polysiloxane having dimethyl hydrogen siloxy groups at both terminals of its molecular chain, and a dimethyl siloxane-methyl hydrogen siloxane copolymer having dimethyl hydrogen siloxy groups at both terminals of its molecular chain. A mixture of two or more selected from the above-described homopolymers and copolymers may be used for the preparation of the surface treatment agent (X).

Examples of a D unit source of the silicone resin comprising the D unit, as a preferable embodiment of the silicone resin (A2) having rubber properties, include dimethyl disilanol, dimethyldimethoxysilane, dimethyldietoxysilane, tetramethyl disiloxane, dimethyl siloxane oligomer, methacryloyloxypropyldimethoxymethylsilane, methyldimethoxyphenylsilane, dietoxymethylphenylsilane, methylphenyl disilanol, 1,4-bis(methyldimethoxysilyl)benzene, and 1,4-bis(methyldietoxysilyl)benzene and the like, and a polymer thereof corresponds to one example of the silicone resin (A2) comprising the D unit.

The weight-average molecular weight of the silicone resin (A2) is not particularly restricted; however, it is usually in a range of 3,000 to 70,000, preferably in a range of 3,500 to 65,000. The vinyl equivalent of the silicone resin (A1) is not particularly restricted; however, it is usually in a range of 0.3 to 10 mol in terms of a SiH group per mol of a vinyl group, preferably in a range of 0.5 to 5 mol. The weight-average molecular weight of the silicone resin (A1) is not particularly restricted; however, it is usually in a range of 6,000 to 45,000, preferably in a range of 6,500 to 40,000. The weight-average molecular weight is measured by GPC (gel permeation chromatography) and is a value in terms of polystyrene.

### <Silicon-Comprising Inorganic Oxide (B)>

The silicon-comprising inorganic oxide (B) is a substance constituted from silicon (Si), oxygen (O), and the like, and composed of one other than an organic compound, and the type of the silicon-comprising inorganic oxide is not restricted. Silica constituted by silicon dioxide is also encompassed in the silicon-comprising inorganic oxide, and may be crystalline silica or non-crystalline silica. The crystalline silica refers to a solid substance having a crystal structure in which atoms constituting a crystal are arranged with three-dimensional regular periodicity to form a space lattice. Examples of the crystalline silica include quartz, cristobalite, and tridymite and the like. The non-crystalline silica refers to a substance in which atoms are not arranged with regular periodicity and are collected without formation of any certain crystal structure. Examples of the non-crystalline silica include glass, silica gel, fumed silica, and diatomaceous earth and the like. The silicon-comprising inorganic oxide (B) may also be a compound comprising a metal element such as sodium, lithium, calcium, magnesium, or zirconium and the like in a constituent component, and examples thereof include sodium silicate, calcium silicate, magnesium silicate, and zircon and the like.

The average particle size of the silicon-comprising inorganic oxide (B) is not particularly restricted; however, it is usually in a range of 1 nm to 50 µm, preferably in a range of 5 nm to 1 µm. The measurement method of the average particle size can be performed with, for example, an electron microscope to provide an average value of the particle sizes of 20 or more of the inorganic oxides (B). In the case of an inorganic oxide having a large aspect ratio, a longer size is used for average particle size measurement.

The surface treatment agent (X) according to the present embodiment is not particularly restricted in terms of the production method thereof, and can be produced by mixing the silicone resin (A), the silicon-comprising inorganic oxide (B), a solvent, a curing agent (C), and an additive.

A solvent comprised in the surface treatment agent (X) is not particularly restricted, and examples thereof include organic solvents such as alcohols, acetonitrile, 2-butoxyethanol, propylene glycol monomethyl ether, benzene, ethylbenzene, toluene, xylene, cyclohexane, methyl acetate, 2-ethoxyethyl acetate, ethyl acetate, butyl acetate, acetone, methyl ethyl ketone, and methyl isobutyl ketone and the like; and mixtures of these organic solvents and water.

### <Curing Agent (C)>

A curing agent comprised in the surface treatment agent (X) is not particularly restricted as long as it can serve as a curing agent of the silicone resin (A), and examples thereof include a curing agent comprising a metal element selected from titanium, platinum, rhodium, and palladium. In particular, a platinum compound is preferred, and examples of the platinum compound include simple metals of platinum group, such as platinum (including platinum black), rhodium, and palladium and the like; platinum chloride, chloroplatinic acid and chloroplatinates, such as H₂PtCl₄·nH₂O, H₂PtCl₆·nH₂O, NaHPtCl₆·nH₂O, KHPtCl₆·nH₂O, Na₂PtCl₆·nH₂O, K₂PtCl₄·nH₂O, PtCl₄·nH₂O, PtCl₂, and Na₂HPtCl₄·nH₂O (wherein n is an integer of 0 to 6, preferably 0 or 6) and the like; alcohol-modified chloroplatinic acid (reaction product of an alcohol and chloroplatinic acid); complexes of chloroplatinic acid with olefins; compounds in which a platinum group metal, such as platinum black or palladium and the like, is supported on a carrier such as alumina, silica, or carbon and the like; rhodium-olefin complexes; chlorotris(triphenylphosphine)rhodium (Wilkinson's catalyst); complexes of platinum chloride, chloroplatinic acid or chloroplatinate with vinyl-comprising siloxane; and compounds in which platinum chloride is supported on a polystyrene-polyethylene glycol; and the like.

These curing agents may be used singly, or in combination of two or more thereof in the preparation of the surface treatment agent (X).

### <Other Additives>

The surface treatment agent (X) according to the present embodiment may also comprise various additives as required. Examples of the additives include a solvent, an electroconductive compound, a surfactant, an antifoaming agent, a leveling agent, a thickening agent, an antibacterial and antifungal agent, a colorant, and a fluorine resin and the like, but these are not limited thereto. These additives may be added within a range that does not impair the effects of the present invention.

The content ratio of each component in the surface treatment agent (X) is not particularly restricted as long as an infrared absorption spectrum of the surface treatment coating formed from the surface treatment agent (X) is a predetermined infrared absorption spectrum, however, the mass ratio of the silicone resin (A) is preferably in a range of 70.0 to 99.5% by mass, more preferably in a range of 80.0 to 99.0% by mass with respect to a total solid content in the surface treatment agent (X).

The mass ratio of the silicon-comprising inorganic oxide (B) is preferably in a range of 0.3 to 30.0% by mass, more preferably in a range of 0.7 to 20.0% by mass with respect to a total solid content in the surface treatment agent (X).

The mass ratio of the curing agent (C) is preferably in a range of 0.005 to 0.045% by mass, more preferably in a range of 0.007 to 0.040% by mass with respect to a total solid content in the surface treatment agent (X).

In a case where the silicone resin (A) comprises the silicone resin (A1) and the silicone resin (A2), the content ratio (mass) is not particularly restricted and (A1):(A2) is preferably in a range of 1:5 to 10:1, more preferably in a range of 1:2 to 5:1.

### <Surface Treatment Agent (Y)>

The surface treatment agent (Y) according to the present embodiment comprises at least an amino group-comprising compound (D). The surface treatment agent (Y) can be used to form a base coating for enhancing burn resistance (in particular, protein- or lipid-derived burn) and/or adhesion of a surface treatment coating to be provided on an objective material of the surgical electrode.

The amino group-comprising compound (D) is not particularly restricted, the amino group may be, for example, any of a primary amino group, a secondary amino group and a tertiary amino group, and the amino group-comprising compound may be one which has two or more of these amino groups. Specifically, the amino group-comprising compound may be, for example, an amine-based curing agent; a homopolymer of a glycidylamine-type epoxy resin, a homopolymer of a polyethyleneimine resin, a homopolymer of a melamine resin, a homopolymer of an aromatic amine resin or the like, or a copolymer comprising these polymers; or an amino group-comprising silane coupling agent and the like. Examples of the amine-based curing agent include dicyandiamide, diethylenetriamine, N-aminoethylpiperazine, m-phenylenediamine, 2-methylimidazole, and 2-ethyl-4-methylimidazole and the like, but these are not limited thereto. In the case of using an amine-based curing agent, it is preferred to use it in combination with an epoxy resin.

The amino group-comprising silane coupling agent is not particularly restricted as long as it has one amino group, and examples thereof include N-2-(aminoethyl)-3-aminopropyldimethylmethoxysilane, N-2-(aminoethyl)-3-aminopropylmethyldimethoxysilane, N-2-(aminoethyl)-3-aminopropyltrimethoxysilane, N-2-(aminoethyl)-3-aminopropyldietylethoxysilane, N-2-(aminoethyl)-3-aminopropylethyldietoxysilane, N-2-(aminoethyl)-3-aminopropyltriethoxysilane, 3-aminopropyldimethylmethoxysilane, 3-aminopropylmethyldimethoxysilane, 3-aminopropyltrimethoxysilane, 3-aminopropyldietylethoxysilane, 3-aminopropylethyldietoxysilane, 3-aminopropyltriethoxysilane, N-phenyl-3-aminopropyltrimethoxysilane, and 3-triethoxysilyl-N-(1,3-dimethylbutylidene)propylamine and the like.

A solvent comprised in the surface treatment agent (Y) is not particularly restricted, and examples thereof include organic solvents, such as alcohols, acetone, acetonitrile, benzene, cyclohexane, methyl acetate, ethyl acetate, and methyl ethyl ketone and the like; and mixtures of these organic solvents and water; and the like. As an organic solvent, an alcohol having not more than 5 carbon atoms is preferred. When the solvent is a mixture of an organic solvent and water, the mass ratio of water comprised in the mixture is preferably less than 5% by mass; however, it is more preferred that the solvent comprises substantially no water.

Further, the surface treatment agent (Y) may also comprise additives, such as an electroconductive compound used for imparting electroconductivity, a leveling agent used for improving the wettability, a film-forming aid used for improving the film-forming properties, an organic or inorganic crosslinking agent used for obtaining a more rigid coating, an antifoaming agent used for inhibiting foam formation, a thickening agent used for controlling the viscosity, and a rust inhibitor and the like, and these additives may be incorporated within a range that does not impair the effects of the present invention.

The total content of the amino group-comprising compound (D) in the surface treatment agent (Y) is not particularly restricted; however, it is preferably in a range of 0.1% by mass to 10% by mass, more preferably in a range of 0.5% by mass to 5% by mass with respect to a total amount of the surface treatment agent (Y).

### <Surgical Electrode Having Surface Treatment Coating and Production Method Thereof>

The surgical electrode having a surface treatment coating according to the present embodiment can be produced by, for example, the following method. By performing first a contacting step of contacting the surface treatment agent (Y) with or over a surface (a part or the entirety of an end portion at least) of a molded surgical electrode, and a drying step of drying the surface treatment agent (Y) brought into contact with the surface of the surgical electrode, as required, a surgical electrode having a base coating can be produced.

Prior to the step of contacting the surface treatment agent (Y) with the surgical electrode, for the purposes of forming irregularities on the surface of the surgical electrode and removing oil, dirt and oxide coatings adhering to the surface of the surgical electrode, a pretreatment may be performed on a metal material. A method for this pretreatment is not particularly restricted, and examples thereof include: a roughening treatment, such as a shot blasting treatment, an etching treatment using a solution (e.g., an acidic solution or an alkaline solution), a grinding treatment, a plasma treatment, or a corona discharge treatment and the like; a washing treatment, such as hot-water washing, solvent washing, alkali degreasing, or acid pickling and the like; an oxide coating removing treatment, and a water-washing treatment; and the like. These treatments may be performed singly, or two or more thereof may be performed in combination.

As a method for contacting the surface treatment agent (Y), a variety of contact methods can be employed, and an optimum method can be selected as appropriate in accordance with, for example, the shape of the surgical electrode. Specific examples of the contact method include a coating method using a coating apparatus, an immersion treatment method, a spray treatment method, a pouring method, a roll coating method, and a bar coating method and the like, but these are not limited thereto.

Further, examples of a method of drying the surface treatment agent (Y) include a method of drying the surface treatment agent (Y) using a hot-air or induction heater, or with infrared ray, near-infrared ray or the like; and a method of drying the surface treatment agent (Y) by vacuum distillation and the like, but these are not limited thereto. The drying temperature is not particularly restricted; however, it is preferably in a range of 40 to 250°C, more preferably in a range of 60 to 180°C. The drying time is also not particularly restricted, and may be changed as appropriate in accordance with, for example, the types of the materials to be used, and the amount of the surface treatment agent (Y) adhering on or over the surface of the surgical electrode.

The method of producing the surgical electrode comprising a surface treatment coating according to the present embodiment further includes: a step of contacting the surface treatment agent (X) with a part or the entirety of the surface of the surgical electrode or a base coating formed on the surgical electrode, and a step of forming a surface treatment coating by drying the surface treatment agent (X) brought into contact with the surgical electrode or the base coating. By performing these steps, a laminate coating that includes the surface treatment coating or the base coating and the surface treatment coating in this order can be formed on the surgical electrode.

As a contact method of the surface treatment agent (X), a variety of contact methods can be employed, and an optimum method can be selected as appropriate in accordance with, for example, the shape of the surgical electrode to be treated. Specific examples of the contact method include coating methods, such as an immersion treatment method, a spray treatment method, a pouring method, a roll coating method, and a bar coating method and the like; and coating methods using one or more coating apparatuses, such as a spin coater, a slit coater, a die coater, a blade coater, and a dispenser or the like.

The drying temperature of the surface treatment agent (X) is not particularly restricted; however, it is preferably in a range of 40 to 250°C, more preferably in a range of 60 to 180°C. A drying method is not particularly restricted, and examples thereof include a method of drying the surface treatment agent (X) using a hot-air or induction heater, or with infrared ray, near-infrared ray or the like; and a method of drying the surface treatment agent (X) by vacuum distillation and the like. The drying time is also not particularly restricted, and can be set as appropriate in accordance with, for example, the types of the materials to be used, and the amount of the surface treatment agent (X) adhering on or over the surface of the surgical electrode. The drying time may be, for example, 10 minutes or longer, or 15 minutes or longer, but 60 minutes or shorter, or 30 minutes or shorter.

Prior to the step of contacting the surface treatment agent (X) with the surgical electrode, for the purposes of forming irregularities on the surface of the surgical electrode and removing oil, dirt and oxide coatings adhering to the surface of the surgical electrode, a pretreatment may be performed on a metal material. A method for this pretreatment is not particularly restricted, and examples thereof include: a roughening treatment, such as a shot blasting treatment, an etching treatment using a solution (e.g., an acidic solution or an alkaline solution), a grinding treatment, a plasma treatment, or a corona discharge treatment and the like; a washing treatment, such as hot-water washing, solvent washing, alkali degreasing, or acid pickling; an oxide coating removing treatment, and a water-washing treatment. These treatments may be performed singly, or two or more thereof may be performed in combination.

By performing the treatments of the surface treatment agent (Y) and the surface treatment agent (X), a surface treatment coating that comprises the base coating and the surface treatment coating in this order can be formed on the surgical electrode. The part where the base coating is formed and the part where the surface treatment coating is formed may be in the same region or different regions. It is noted here that the base coating exists as an underlayer in the part where the surface treatment coating is formed.

In one example, the base coating formed by the surface treatment agent (Y) may be formed on the entire surface of the blade part of the end portion 11 as illustrated with hatching in FIG. 7(a) or on the blade part of the end portion 11 and a part of the intermediate connecting part as illustrated with hatching in FIG. 7(b), or may be formed on a part away from the electrical connection portion 13 in the blade part of the end portion 11 as illustrated in FIG. 7(c) (the base coating is not formed on a part close to the intermediate connecting part).

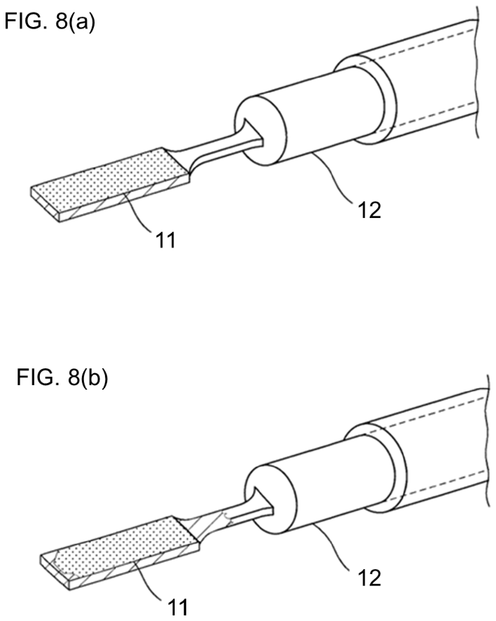

In another example, the surface treatment coating formed by the surface treatment agent (X) may be formed on the entire surface of the flat part (both the upper surface and the lower surface of the blade part) in the end portion as illustrated with a dot pattern in FIG. 8(a), and the surface treatment coating is not formed on the side surfaces (surfaces other than the flat part) of the blade part, but the surface treatment coating may be formed in a region of 2 mm to the distal end from the electrical connection portion 13 side, the region corresponding to the side surfaces in a longitudinal direction of the blade part. In yet another example, as illustrated with a dot pattern in FIG. 8(b), the surface treatment coating is not formed on neither the side surfaces of the blade part nor the distal ends of the upper and the lower surfaces. In this manner, by not forming the surface treatment coating on the side surfaces as well as the distal ends, particularly the corners of the upper and the lower surfaces (parts away from the electrical connection portion 13), a high frequency can be sufficiently emitted from the part of the end portion that can come into close contact with a living tissue and the like, namely a part that discharges electricity (hereinafter, referred to as "discharging part"); therefore, a surgical electrode instrument can be ensured to have good incision capability. In cases where the surface treatment coating is formed on the side surfaces as well as the distal ends, particularly the corners of the upper and the lower surfaces, it is preferred to control the coating thickness to be small (e.g., 10 µm or less, preferably 5 µm or less, more preferably 2 µm or less). By this, a high frequency can be sufficiently emitted from the discharging part that can come into close contact with a living tissue, so that deterioration of the incision capability can be inhibited.

In the end portion having the surface treatment coating (a blade part in the case of a blade-type surgical electrode), the coating amount of the coating composed of the surface treatment agent (Y) is not particularly restricted; however, it is preferably in a range of 0.1 mg/m² to 50 mg/m², more preferably in a range of 1 mg/m² to 40 mg/m². When the base coating is formed from an amino group-comprising silane coupling agent, it is preferred that the coating amount be in the above-described range in terms of SiO₂-equivalent mass.

The amount of the base coating can be determined by measuring the coating amount on a metal material having a prescribed area. Further, the base coating is formed from an amino group-comprising silane coupling agent, the coating amount can be determined by analyzing the base coating by a fluorescent X-ray method, calculating the mass in terms of SiO₂ from the Si intensity, and then determining the coating amount per unit area.

Moreover, the total coating thickness of the base coating (surface treatment agent (Y)) and the surface treatment coating (surface treatment agent (X)), which are formed on the end portion (a blade part in the case of a blade-type surgical electrode), is preferably in a range of 10 µm to 400 µm, more preferably in a range of 20 µm to 300 µm, still more preferably in a range of 30 µm to 200 µm, particularly preferably in a range of 50 µm to 150 µm.

### EXAMPLES

The actions and effects of the present invention will now be described concretely by way of Examples. It is noted here, however, that the following descriptions of Examples do not restrict the scope of the present invention by any means.

### (1) Preparation of Surgical Electrodes

Blade-type surgical electrodes having a plate-form end portion 11 as illustrated in FIG. 1 were prepared. The material and the blade part size of each of the thus prepared surgical electrodes are shown below. The surface roughness (arithmetic average roughness: Ra) of the blade part was measured using a three-dimensional surface roughness analyzer (model: SURFCOM 570A, manufactured by TOKYO SEIMITSU CO., LTD.). The measurement was performed by 2.0-mm scanning at a rate of 0.3 mm/s.
(Z1) Material of surgical electrode: stainless steel SUS304 Ra = 0.14 µm
   Size of blade part: 0.3 mm in plate thickness, 17.0 mm in length, 2.5 mm in width
(Z2) Material of surgical electrode: stainless steel SUS316L Ra = 0.14 µm
   Size of blade part: 0.3 mm in plate thickness, 17.0 mm in length, 2.5 mm in width
(Z3) Material of surgical electrode: tungsten steel Ra = 0.14 µm
   Size of blade part: 0.3 mm in plate thickness, 17.0 mm in length, 2.5 mm in width

The blade part of each surgical electrode was immersed in 2-propanol (special grade, manufactured by Junsei Chemical Co., Ltd.), and ultrasonicated for 10 minutes to remove oil and dirt from the surface. Subsequently, the blade part was dried at 120°C for 10 minutes to remove 2-propanol adhering thereto.

### (2) Preparation of Surface Treatment Agents

In the preparation of surface treatment agents (X), surface treatment agents X1 to X22 were prepared by mixing the following silicone resin (A), silicon-comprising inorganic oxide (B), and curing agent (C) with xylene such that the resulting solutions had a solid mass concentration of 15 to 40%. In Table 1, the values in the columns of "% by mass" under "Silicone resin (A)", "Silicon-comprising inorganic oxide (B)", and "Curing agent (C)" each indicate the mass ratio of each component with respect to a total solid content of these components. Further, the ratio of A1:A2 in each of surface treatment agents X2 to X17 indicates the solid content mass ratio.

### [Silicone Resins (A)]

A1: mixture of polydimethyl siloxane having dimethylvinyl siloxane group at each of both terminals of its molecular chain, and methyl hydrogen siloxane-dimethyl siloxane copolymer having trimethylsilyl group at each of both terminals of its molecular chain (T unit)
A2: two-component addition type liquid silicone rubber having vinyl group in terminal group (D unit)
A3: one-component curing type oligomer (manufactured by Shin-Etsu Chemical Co., Ltd., trade name = KR-400) (T unit)
A4: methyl/phenyl-based silicone resin (manufactured by Shin-Etsu Chemical Co., Ltd., trade name = KR-282) (D unit and T unit)
A5: methyl/phenyl-based silicone resin (manufactured by Shin-Etsu Chemical Co., Ltd., trade name = KR-300) (D unit and T unit)

### [Silicon-Comprising Inorganic Oxides (B)]

B1: non-crystalline silica (average particle size 12 nm)
B2: crystalline silica (average particle size 12 nm)
B3: sodium silicate (average particle size 50 nm)
B4: calcium silicate (average particle size 40 nm)
B5: magnesium silicate (average particle size 30 nm)
B6: zircon (average particle size 1 µm)

### [Curing Agents (C)]

C1: tetrachloroplatinate(II) (manufactured by Shin-Etsu Chemical Co., Ltd., trade name = D-168)
C2: titanium butoxide (manufactured by Shin-Etsu Silicone, trade name = D-20)
C3: titanium acetylacetonate (manufactured by Matsumoto Fine Chemical Co., Ltd., trade name = TC-100)
C4: platinum-based catalyst (manufactured by ARAKAWA CHEMICAL INDUSTRIES, LTD., trade name = CATA93B)

As the surface treatment agent (Y), a solution (Y1 or Y2) was prepared by mixing D1 or D2 as the following amino group-comprising compound (D) with ethanol such that D1 or D2 had a solid mass concentration of 1.0%.

### [Amino Group-Comprising Compounds (D)]

D1: 3-aminopropyltriethoxysilane
D2: N-(2-aminoethyl)-3-aminopropyltrimethoxysilane

**[Table 1]**

| Surface treatment agent (X) | Silicone resin (A) | | Silicon-comprising inorganic oxide (B) | | Curing agent | |
|---|---|---|---|---|---|---|
| | Type | % by mass | Type | % by mass | Type | % by mass |
| X1 | A1 | 88.576 | B1 | 11.405 | C1 | 0.019 |
| X2 | A1 : A2 = 2 : 1 | 92.739 | B1 | 7.241 | C1 | 0.020 |
| X3 | A1 : A2 = 1 : 1 | 94.659 | B1 | 5.322 | C1 | 0.019 |
| X4 | A1 : A2 = 1 : 2 | 96.512 | B1 | 3.471 | C1 | 0.017 |
| X5 | A1 : A2 = 1 : 2.5 | 97.046 | B1 | 2.936 | C1 | 0.018 |
| X6 | A1 : A2 = 1 : 3 | 97.396 | B1 | 2.583 | C1 | 0.021 |
| X7 | A1 : A2 = 1 : 3.5 | 97.711 | B1 | 2.271 | C1 | 0.018 |
| X8 | A1 : A2 = 1 : 4 | 97.936 | B1 | 2.047 | C1 | 0.017 |
| X9 | A1 : A2 = 1 : 5 | 98.271 | B1 | 1.711 | C1 | 0.018 |
| X10 | A1 : A2 = 1 : 1 | 94.659 | B2 | 5.322 | C1 | 0.019 |
| X11 | A1 : A2 = 1 : 1 | 94.659 | B3 | 5.322 | C1 | 0.019 |
| X12 | A1 : A2 = 1 : 1 | 94.659 | B4 | 5.322 | C1 | 0.019 |
| X13 | A1 : A2 = 1 : 1 | 94.659 | B5 | 5.322 | C1 | 0.019 |
| X14 | A1 : A2 = 1 : 1 | 94.659 | B6 | 5.322 | C1 | 0.019 |
| X15 | A1 : A2 = 1 : 1 | 94.659 | B1 | 5.322 | C2 | 0.019 |
| X16 | A1 : A2 = 1 : 1 | 94.659 | B1 | 5.322 | C3 | 0.019 |
| X17 | A1 : A2 = 1 : 1 | 94.659 | B1 | 5.322 | C4 | 0.019 |
| X18 | A1 | 73.587 | B1 | 26.396 | C1 | 0.016 |
| X19 | A2 | 100.000 | - | - | - | - |
| X20 | A3 | 100.000 | - | - | - | - |
| X21 | A4 | 86.000 | B1 | 14.000 | - | - |
| X22 | A5 | 86.000 | B1 | 14.000 | - | - |

### (3) Production of End Portion 11 Having Surface Treatment Coating

On the flat parts (both surfaces) of the end portion 11, each surface treatment agent (X) shown Table 2 was applied using the below-described dispenser and subsequently dried for 30 minutes at the drying temperature, whereby surgical electrodes of Examples 1 to 27 and Comparative Examples 1 to 4, each of which had a surface treatment coating of a prescribed thickness (see Table 2), were obtained.

Dispenser (desktop-type robot): manufactured by Musashi Engineering, Inc., trade name: ML-808GX, SM4000MEGAX-3A-SS

Further, the same treatment was performed on the flat parts (both surfaces) of the end portion 11 having a base coating as required. In the case of the base coating, the blade part removed of oil and dirt was immersed in each surface treatment agent (Y). After the immersion, the blade part was dried at 120°C for 10 minutes to obtain a surgical electrode comprising a base coating. In those cases where the base coating was formed using any one of Y1 and Y2, the base coating was analyzed by a fluorescent X-ray method, and the mass was calculated in terms of SiO₂ from the Si intensity to determine the coating amount per unit area.

**[Table 2]**

| Example/ Comparative Example | Surgical electrode | First coating | | | Second coating | | |
|---|---|---|---|---|---|---|---|
| | | Surface treatment agent (Y) | Coating amount | Drying temperature | Surface treatment agent (X) | Coating amount | Drying temperature |
| | | | mg/m² | °C | | µm | °C |
| Example 1 | Z1 | - | - | - | X1 | 100 | 150 |
| Example 2 | Z1 | Y1 | 10 | 100 | X1 | 100 | 150 |
| Example 3 | Z1 | - | - | - | X2 | 100 | 150 |
| Example 4 | Z1 | - | - | - | X3 | 100 | 150 |
| Example 5 | Z1 | Y1 | 10 | 100 | X3 | 100 | 150 |
| Example 6 | Z1 | - | - | - | X4 | 100 | 150 |
| Example 7 | Z1 | Y1 | 10 | 100 | X4 | 100 | 150 |
| Example 8 | Z1 | - | - | - | X5 | 100 | 150 |
| Example 9 | Z1 | - | - | - | X6 | 100 | 150 |
| Example 10 | Z1 | - | - | - | X7 | 100 | 150 |
| Example 11 | Z1 | - | - | - | X8 | 100 | 150 |
| Example 12 | Z1 | Y1 | 10 | 100 | X8 | 100 | 150 |
| Example 13 | Z1 | Y1 | 10 | 100 | X9 | 100 | 150 |
| Example 14 | Z1 | Y1 | 1 | 100 | X3 | 100 | 150 |
| Example 15 | Z1 | Y1 | 50 | 100 | X3 | 100 | 150 |
| Example 16 | Z1 | Y2 | 10 | 100 | X3 | 100 | 150 |
| Example 17 | Z1 | - | - | - | X10 | 100 | 150 |
| Example 18 | Z1 | - | - | - | X11 | 100 | 150 |
| Example 19 | Z1 | - | - | - | X12 | 100 | 150 |
| Example 20 | Z1 | - | - | - | X13 | 100 | 150 |
| Example 21 | Z1 | - | - | - | X14 | 100 | 150 |
| Example 22 | Z1 | - | - | - | X15 | 100 | 150 |
| Example 23 | Z1 | - | - | - | X16 | 100 | 150 |
| Example 24 | Z1 | - | - | - | X17 | 100 | 150 |
| Example 25 | Z1 | - | - | - | X18 | 100 | 150 |
| Example 26 | Z2 | Y1 | 10 | 100 | X3 | 100 | 150 |
| Example 27 | Z3 | Y1 | 10 | 100 | X3 | 100 | 150 |
| Comparative Example 1 | Z1 | Y1 | 10 | 100 | X19 | 100 | 120 |
| Comparative Example 2 | Z1 | Y1 | 10 | 100 | X20 | 1 | 180 |
| Comparative Example 3 | Z1 | Y1 | 10 | 100 | X21 | 20 | 250 |
| Comparative Example 4 | Z1 | Y1 | 10 | 100 | X22 | 20 | 250 |

### (4) FT-IR Measurement (β_{A}/α_{A}, γ_{A}/α_{A}, γ_{A}/β_{A})

Analysis was performed by an ATR method as one infrared spectroscopy. An apparatus used here was FT-IR (Spectrum Two) manufactured by Perkin Elmer, to which auxiliary equipment for ATR measurement (GladiATR^{™}) was attached. The sample was prepared by treating stainless steel with the surface treatment agent (X), and a sample surface was pressure-bonded to a measurement part to perform measurement. After the measurement, ATR correction and baseline correction were performed and then correction was performed such that the absorbance at 4000 cm⁻¹ was 0. The value of the ratio (β_{A}/α_{A}) of the absorbance (β_{A}) of the peak β to the absorbance (α_{A}) of the peak α was here the average of the values measured three times. The value of the ratio (γ_{A}/α_{A}) of the peak intensity (γ_{A}) of the peak γ to the peak intensity (α_{A}) of the peak α and the value of the ratio (γ_{A}/β_{A}) of the peak intensity (γ_{A}) of the peak γ to the peak intensity (β_{A}) of the peak β were also each the average of the values measured three times in the same manner. The values obtained are shown in Table 3.

### (5) Evaluation Tests

### (5-1) Burn Resistance A (under Assumption of Case Where Blood was Much)

Two droplets (one droplet: 3 µl) of porcine blood (Tokyo Shibaura Zoki Co, Ltd.) were dropped to each of the surgical electrodes, and a cycle of heating at 350°C/1 min and then air cooling at room temperature for 1 minute in a muffle furnace was performed for three sets. After cooling of each of the surgical electrodes at room temperature, the blood attached onto the coating film was dry-wiped out with KimWipes, two droplets of the porcine blood were again dropped onto a coating film, and the cycle of heating at 350°C/1 min and then air cooling at room temperature for 1 minute was performed for three sets. Finally, each of the surgical electrodes was cooled to room temperature and then dry-wiped off with KimWipes. The residual ratio of the burn area of blood after the last dry-wiping-off to the burn area of blood on each of the surgical electrodes after completion of the third set in the second cycle was determined, and the burn resistance was evaluated based on the following criteria. The results thereof are shown in Table 3.
S: The residual ratio was less than 1%
A: The residual ratio was 1% to less than 15%
B: The residual ratio was 15% to less than 30%
C: The residual ratio was 30% to less than 45%
D: The residual ratio was 45% or more, or peeling of the coating was present

### (5-2) Burn Resistance B (under Assumption of Case Where Protein and/or Lipid were/was Much)

The thus obtained surgical electrodes of Examples 1 to 27 and Comparative Examples 1 to 4 were each electrically connected to the below-described electrosurgical instrument main body. Further, a counter electrode plate electrically connected to the electrosurgical instrument main body was attached to a stainless-steel container in which a porcine liver was placed.

### <Electrosurgical Instrument Main Body (High-Frequency Apparatus and Control Pencil)>

High-frequency apparatus: Excalibur PLUS PC, medical device approval No.: 20700BZY01171

Control pencil: manufactured by Japan Medicalnext Co., Ltd., disposable control pencil, medical device approval No.: 20300BZY01003000

The electrosurgical instrument main body was operated in the pure cutting mode (output: 30 W), and the blade part was inserted in the vertical direction at an angle of 45° with respect to the porcine liver surface, and at a depth of 12 mm, the thus inserted blade part was moved by 60 mm parallel to the porcine liver surface at a speed of 20 mm/s. Each surgical electrode used for the two repeated cutting operations was cooled to room temperature, and the evaluation part was subsequently held with fingers through a piece of gauze and wiped once. Thereafter, the coating of the evaluation part was visually observed, and the burn resistance was evaluated based on the following evaluation criteria. The results thereof are shown in Table 3.
S: The remaining burn occupied less than 1% with respect to the evaluation part.
A: The remaining burn occupied 1% to less than 5% with respect to the evaluation part.
B: The remaining burn occupied 5% to less than 15% with respect to the evaluation part.
C: The remaining burn occupied 15% or more with respect to the evaluation part.

### (5-3) Adhesion

The electrosurgical instrument main body was operated in the pure cutting mode (output: 30 W), and the blade part was inserted in the vertical direction at an angle of 45° with respect to the porcine liver surface, and at a depth of 12 mm, the thus inserted blade part was moved by 60 mm parallel to the porcine liver surface at a speed of 20 mm/s. Each surgical electrode used for the two repeated cutting operations was cooled to room temperature, and the evaluation part was subsequently held with fingers through a piece of gauze and wiped once. Thereafter, the coating of the evaluation part was visually observed, and the burn resistance was evaluated based on the following evaluation criteria. The results thereof are shown in Table 3.
S: The peeled area of the coating was less than 1% with respect to the evaluation part.
A: The peeled area of the coating was 1% to less than 5% with respect to the evaluation part.
B: The peeled area of the coating was 5% to less than 15% with respect to the evaluation part.
C: The peeled area of the coating was 15% or more with respect to the evaluation part.

**[Table 3]**

| Example/ Comparative Example | FT-IR analysis | | | | Evaluation test | | |
|---|---|---|---|---|---|---|---|
| | β_{A}/α_{A} | γ_{A}/α_{A} | γ_{A}/β_{A} | γ | Burn resistance A | Evaluation by pure cutting mode (30 W) | |
| | | | | | | Burn resistance B | Adhesion |
| Example 1 | 0.870 | 0.067 | 0.077 | ○ | A | A | S |
| Example 2 | 0.870 | 0.067 | 0.077 | ○ | A | S | S |
| Example 3 | 0.941 | 0.049 | 0.052 | ○ | A | S | S |
| Example 4 | 0.967 | 0.038 | 0.039 | ○ | S | S | S |
| Example 5 | 0.967 | 0.038 | 0.039 | ○ | S | S | S |
| Example 6 | 0.995 | 0.026 | 0.026 | ○ | S | S | S |
| Example 7 | 0.995 | 0.026 | 0.026 | ○ | S | S | S |
| Example 8 | 1.005 | 0.024 | 0.024 | ○ | S | S | S |
| Example 9 | 1.011 | 0.021 | 0.021 | ○ | S | S | S |
| Example 10 | 1.017 | 0.022 | 0.021 | ○ | S | S | S |
| Example 11 | 1.024 | 0.020 | 0.019 | ○ | S | A | A |
| Example 12 | 1.024 | 0.020 | 0.019 | ○ | S | S | S |
| Example 13 | 1.035 | 0.018 | 0.018 | ○ | A | B | B |
| Example 14 | 0.967 | 0.038 | 0.039 | ○ | S | A | A |
| Example 15 | 0.967 | 0.038 | 0.039 | ○ | S | A | A |
| Example 16 | 0.967 | 0.038 | 0.039 | ○ | S | S | S |
| Example 17 | 0.967 | 0.038 | 0.039 | ○ | S | S | S |
| Example 18 | 0.967 | 0.038 | 0.039 | ○ | S | S | S |
| Example 19 | 0.967 | 0.038 | 0.039 | ○ | S | S | S |
| Example 20 | 0.967 | 0.038 | 0.039 | ○ | S | S | S |
| Example 21 | 0.967 | 0.038 | 0.039 | ○ | S | S | S |
| Example 22 | 0.967 | 0.038 | 0.039 | ○ | S | S | S |
| Example 23 | 0.967 | 0.038 | 0.039 | ○ | S | S | S |
| Example 24 | 0.967 | 0.038 | 0.039 | ○ | S | S | S |
| Example 25 | 0.778 | 0.165 | 0.212 | ○ | B | B | B |
| Example 26 | 0.967 | 0.038 | 0.039 | ○ | S | S | S |
| Example 27 | 0.967 | 0.038 | 0.039 | ○ | S | S | S |
| Comparative Example 1 | 1.110 | - | - | X | S | C | C |
| Comparative Example 2 | 0.596 | - | - | X | D | C | C |
| Comparative Example 3 | 0.474 | 0.289 | 0.611 | ○ | C | C | S |
| Comparative Example 4 | 0.590 | 0.219 | 0.371 | ○ | D | C | S |

The present invention has been described above in detail referring to concrete examples thereof; however, it is obvious to those skilled in the art that various modifications and changes can be made without departing from the gist and the scope of the present invention.

### EXPLANATION OF REFERENCES

10, 20, 30, 40, 60: Surgical electrode
11, 21, 31, 41, 61: End portion
12: Intermediate portion
13, 23, 33, 43: Electrical connection portion
14, 24, 34, 44: Cover

## Claims

1. A surgical electrode of an electrosurgical instrument used for surgery of a living tissue,
wherein the surgical electrode comprises an end portion capable of emitting a high frequency,
the end portion has a surface treatment coating, and
the surface treatment coating has a peak (α) in a range of 950 cm⁻¹ to 1060 cm⁻¹, a peak (β) in a range of 720 cm⁻¹ to 830 cm⁻¹, and a peak (γ) in a range of 450 cm⁻¹ to 495 cm⁻¹ in an infrared absorption spectrum, and a ratio (β_{A}/α_{A}) of a peak intensity (β_{A}) of the peak β to a peak intensity (α_{A}) of the peak α is in a range of 0.770 to 1.040.

2. The surgical electrode according to claim 1, wherein the surface treatment coating comprises a silicone resin (A), and the silicone resin (A) comprises a constituting unit composed of a D unit and a constituting unit composed of a T unit.

3. The surgical electrode according to claim 2, wherein the surface treatment coating comprises a silicon-comprising inorganic oxide (B).

4. The surgical electrode according to any one of claims 1 to 3, wherein the end portion further comprises a base coating, which is formed from a surface treatment agent (Y) comprising an amino group-comprising compound (D).
